# EUROPEAN PATENT SPECIFICATION

(11) **EP 1 017 839 B1**
(45) Date of publication and mention of the grant of the patent: **20.10.2004**
(21) Application number: 98940758.0
(22) Date of filing: 26.08.1998
(51) Int. Cl.: C12N 15/87, C12M 3/02, C12N 5/10

(54) **METHOD OF INTRODUCING ORGANIC MOLECULES INTO TARGET CELLS**
METHODE ZUR EINFÜHRUNG ORGANISCHER MOLEKÜLE IN ZIELZELLEN
PROCEDE D'INTRODUCTION DE MOLECULES ORGANIQUES DANS DES CELLULES CIBLES

(30) Priority: 26.08.1997 SE 9703073
(43) Date of publication of application: 12.07.2000
(73) Proprietor: Avaris AB, 171 77 Stockholm (SE)
(72) Inventor: SMITH, Edvard, S-129 38 Hägersten (SE); BRANDEN, Lars, S-116 61 Stockholm (SE)
(74) Representative: Larfeldt, Helene
(86) International application number: PCT/SE1998/001527
(87) International publication number: WO 1999/010517

(56) References cited:
- WO-A2-95/10619
- DIALOG INFORMATION SERVICES, File BIOSIS, Dialog Accession No. 11281083, Biosis Accession No. 199800062415, BRANDEN LARS et al., "Reverse Flow-Through Transduction: A New Method for Gene Delivery"; & BLOOD, 90 (10 Suppl. 1 Part 2), 15 Nov. 1997, p. 409B.

## Description

### Field of the invention

The present invention relates to a method of introducing organic molecules into target cells, which comprises the step of passing a liquid flow comprising said organic molecules through said target cells. In addition, the present invention also relates to a system especially adapted for performing the method according to the invention.

### Background and prior art

Since the discovery of the DNA molecules by Watson and Crick in the 50's, huge scientific progress has been made within the field of biochemistry and biology. Recombinant DNA techniques and techniques for genetic manipulation are continuously developped based on the new discoveries and new medical applications thereof are proposed every day. In general, such methods are based on the principle that some kind of genetic information containing material, such as a gene, is transferred from its original and native environment to a new environment, where some desired properties thereof may be utilized in a new context. Usually, some kind of carrier or vector is used for such a transfer procedure, of which one example is virus. Viruses are due to the native properties thereof capable of infecting cells and thereby transferring its own contents of nucleic acids thereto. Successful genetic manipulation requires appropriate reagents, such as vectors, as well as practical and effective procedures for the performance thereof. As one example of a medicinal application, which is of utmost interest at the moment, may gene therapy be mentioned. In gene therapy, vectors, such as virus particles, are used to introduce nucleic acid sequences in receptor cells, where said sequences may compensate for damages or mutations, or even correct mutations, in the native genes of said cells.

During the elaboration of better and more efficient recombinant techniques, it has appeared that the cells which are desirable as receptor or target cells often are quite difficult to infect. The simplest and most straight forward approach to enhance the number of infected cells is to increase the amount of virus used during the infection procedure, whereby the number of completed infection occurences in receptor cells hopefully increase as well. The result of this approach is, however, an increased demand for cells producing virus in high concentrations. Problems arise, however, when cells are to produce large amounts of a virus. In many cases, the virus is toxic to the producer cells when present in such massive amounts, and, accordingly, the cells are killed when the virus production is increased. In cases where large foreign nucleic acid sequences have been introduced in the virus, the virus particles may not be assembled in a correct way during a large scale production..

Different ways of transfecting cells with vectors, such as viruses, have been proposed. One procedure is disclosed, where a suspension of target cells is held in a container, whereafter a virus containing cell medium is added thereto. However, since a virus is a colloidal particle, it will move randomly with Brownian motions in a liquid. Accordingly, the transfection takes place randomly in such a procedure, depending on the amount of particles that eventually hit the cells. Only a small fraction of the virus particles will ever reach contact with target cells and, in addition, the contact area and binding achieved is usually too loose to permit a sufficient transfection. The virus containing cell suspension may be stirred or agitated in order to increase the efficacy of such a process. Still, the efficiency of the process is low and not optimal for an industrial method.

In order to increase the contact area between target cells and virus, and thus increase the number of infection occurences, target cells have been immobilized on membranes. Then, a virus containing supernatant is passed through the membrane. WO95/10619 discloses a method wherein a liquid comprising vectors, e.g. retroviruses, is passing stationary target cells on a porous filter and a system comprising a reaction vessel and means for providing a constant flow of said liquid such as tubing and pumping devices enabling recirculation. Indeed, a better contact between virus particles and cells than in the case described above may be obtained. However, such a membrane technique will exhibit other essential drawbacks. One problem when using a membrane in connection with cells is that it will sooner or later be obstructed by clogging cells. Thus, the life-time of such a membrane and also the possible choices of flow conditions are limited. Another problem results from the fact, that the virus containing supernatant must be forced against the target cells on the solid membrane, whereupon the virus binding receptors on the target cells are often damaged. Consequently, the possible binding of virus theron will be reduced. An additional drawback with the membrane techniques originates from the fact that only a limited number of cells may be immobilized on each membrane, which number is restricted by the area of the membrane. In fact, preliminary calculations reveal that to attain a sufficiently large amount of cells for clinical experiments the diameter of such a membrane need to be about 3-4 m. In addition, the amount of supernatant needed to wet such an area is enormous. Naturally, such a large membrane is hardly applicable in practice.

Other cellular filters have been proposed for similar applications. In Biotechnol. Prog. 1990, 6, p. 104-113, by Krishna Mohan et al, a cellular filter is disclosed for the removal of trace viral contaminants in blood. However, the aim of these authors is to purify blood or similar fluids, and therefore, the properties of the resulting fluid are stressed, while no particular account is taken to protect or enhance the cells that absorb the virus. In this publication, one strategy is disclosed, where cells are grown on microcarriers and stabilized by a cross-linking agent. Consequently, a cellular filter is accomplished. Said filter will exhbit properties similar to the above described membranes with immobilized cells thereon, i.e. it will constitute an almost stable element against which cells absorbing virus will be forced. Another strategy disclosed in this article involves the use of clusters or aggregates of cells around microcarriers. The microspheres used to cluster the cells exhibits a magnetic nature, which is then utilized combined with other magnetic equipment around the reaction vessel. Obviously, such a magnetic agitation will be efficient for the application disclosed in the article, i.e. the recovery of a virus free solution. However, in the instance when the infected cells are the products to be used for later applications, it will result in too large a cell desintagration and, in addition, the magnetic field may be harmful as regards internal properties of the absorbing cells.

### Objects of the invention

The object of the present invention is to solve the problems defined above. Accordingly, the present invention relates to a method of introducing organic molecules into target cells, which comprises the step of passing a liquid flow, which comprises particles originally containing said organic molecules, through said target cells. Thereby, the organic molecules are efficiently introduced into the target cells. Another object of the invention is a cell harbouring a foreign organic molecule introduced by the method according to the invention. A last aspect of the invention is a system especially suited for performing the present method.

### Brief description of the drawing

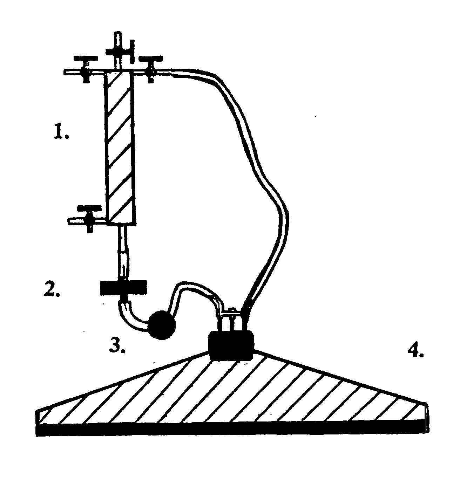

Figure 1 discloses an example of a system according to the invention, which is denoted a reverse flow-through transduction (RFTT) system. It comprises a reaction vessel or chamber, means for separation of undesired material from the liquid flow, a pumping device as well as a vessel for the production of particles comprising organic molecules. In addition, part of the requisite tubing etc are shown.

### Detailed description of the invention

In a first aspect, the present invention relates to a method of introducing organic molecules into target cells, which comprises the step of passing a liquid flow, which comprises particles originally containing said organic molecules, by said target cells, and preferably through a suspension or solution comprising said target cells. The liquid flow is controlled to provide essentially constant flow conditions between liquid comprising said particles containing the organic molecules and target cells floating free in a suspension. As used herein, the term "essentially constant flow" should be interpreted as a flow, which is constant over a period of time, while allowing shorter periods of intermittent flow therein. Thus, the liquid flow is controlled to provide a sufficiently close contact, as regards both contact time and area, between the particles and the target cells, to enable the organic molecules to exit the particles and be introduced efficiently into the target cells. In order to set a sufficient value of the flow, samples of target cells may be taken out and analyzed as regards whether the organic molecules in question have been introduced therein or not. Such methods are well known and may be found in the literature. Thus, such testing or analysis is easily performed by the person skilled within this field.

In the preferred embodiment of the method according to the present invention, the flow conditions are achieved by providing a direction of the liquid flow which essentially counteracts the gravitational force, or any suitable multiplicity thereof, of the target cells. Thus, the flow will be set at a value, which *inter alia* depends on the weight of the target cells, so that stabile and constant flow conditions are achieved. The liquid flow according to the present invention is most preferably passed through the target cells from below, so as to counteract the weight of the target cells. Accordingly, in an advantageous embodiment, the method according to the invention is performed during conditions similar to a fluidized bed, where the floating target cells comprise the bed. Thus, the target cells are free floating, surrounded by liquid comprising particles containing organic molecules. In general, target cells which are able to absorb, or bind to, other cells or particles, exhibit the receptors for that purpose on their surfaces. Accordingly, practically all such receptors on the target cells surfaces, to which particles may bind, will be exposed to the liquid flow and the particles therein. As compared to the prior art techniques, this feature of the present invention will increase the number of bound particles as well as the binding rate and thus enhance the efficacy of the introduction of organic molecules from the particles into target cells.

In alternative embodiments of the present method, a centrifugal force may be applied on the target cells and used instead of the gravitational force used as above, in which case the liquid flow is passed through the target cells in a direction, which essentially counteracts said centrifugal force. As a person skilled in this area easily realizes, there exist several different possibilities and embodiments of the herein examplified concept of allowing a liquid flow to counteract the gravitational or centrifugal force of target particles, all of which are encompassed by the claims defining the present invention.

In addition to the above defined advantageous contact area and time, another feature of the method according to the invention is, that depending on the specific relation between the liquid flow and the weight of the target cells according to the invention, the liquid flow comprising particles which originally contain the organic molecules will apply a certain pressure on the target cells. This pressure will aid in the binding of the particles at the target cell surfaces, thereby further enhancing the process of introducing organic molecules therein. Naturally, said pressure will not be as high as to damage or desintegrate the target cells. Since the target cells are floating free in a suspension or solution during the method according to the invention, there will be no solid member onto which the target cells may be forced, and thereby damaged. Thus, the pressure will enhance the introduction of organic molecules without unnecessary damage on the target cells. Contrary, in the prior an methods, target cells are forced towards solid membranes or filters by a liquid flow. Such solid membranes or filters are in addition easily clogged and accordingly blocked after use during a certain period of time. In summary, the problems of desintegration of cells due to an applied pressure onto a solid member is eliminated by the method according to the present invention, wherein the target cells themselves comprise the only filter used. In addition to this, the method according to the invention may be used as a continuous process during a virtually unlimited period of time, as no clogging can occur.

Accordingly, the method according to the present invention is advantageously used in the context of fragile cells, which otherwise might be impaired by membranes or solid filters used in the prior art methods for introduction of organic molecules.

The constant flow conditions in the method according to the invention are easily set at flow rates suitable for each specific case, depending on both the kind of target cells and the organic material to be introduced therein, or transferred thereto.

In one advantageous embodiment of the present invention, the same liquid flow is passed several times through the target cells. Such a recirculation procedure will increase the total number of target cells, which finally harbour organic molecules, and thus increase the efficacy of the process. Such a recirculation is not comparable to a recirculation over a membrane with immobilized cells according to the prior art, as a membrane will eventually be occluded by particles, which limits the number of times recirculation is possible over such membranes.

In one particular embodiment of the method according to the present invention, the target cells are immobilized on small carriers capable of being fluidized, such as microcarriers. However, said microcarriers are still floating free in the suspension, whereby the same advantages as described above with free floating cells are achieved. This embodiment differs from the strategy disclosed in the previously mentioned article from Biotechnol. Prog, 1990, 6, p 104-113, by the fact that according to the invention, the microcarriers are floating free, as in a fluidized bed, while the microcarriers disclosed in said article are cross-linked and thereby stabilized. Whether it is more advantageous to immobilize the target cells on microcarriers or to use target cells free in a suspension or a solution is easily determined by a person skilled in the art, depending on the kind of target cells as well as other parameters.

The particles that contain organic molecules to be introduced into, or transferred to, target cells may be any particles, carriers or vectors capable of interaction with cells. The particles may be native viruses or viruses, wherein a genetic sequence, such as a marker sequence or a therapy gene, has been introduced by recombinant techniques. Thanks to the advantageous contact area and time between target cells and the particles containing organic molecules, the method according to the invention is especially suitable for the case where the herein defined introduction is a transfection of genetic information from a rapidly degrading virus. For example, retrovirus are e.g. degraded as rapidly as 50% in 8h. Accordingly, such viruses will need to rapidly hit the target cells, bind thereto and infect them. Alternatively, the particle may be any other kind of carrier commonly used in techniques for genetic manipulation or especially designed for a particular purpose.

In its broadest aspect, the method according to the present invention may use any organic molecules capable of interaction with target cells. The organic molecules may be any biomolecules, *inter alia* nucleic acids, complexes of nucleic acids with liposomes, preferably cationic liposomes, such as complexes suitable for use in liposomal transfection procedures, other complexes, or genetic information originating from virus, such as DNA or RNA. The organic molecules may be native, produced by recombinant DNA techniques or synthetically produced, such as by organic synthesis or peptide synthesis. The expression "particles containing organic molecules"as used herein may refer to one molecule such as a peptide or protein, which thus constitutes the particle referred to herein as well as the organic molecule, in one single element. The organic molecules used in the method according to the invention may be molecules containing some kind of genetic information, such as DNA or RNA present in a virus particle. Thus, the method of introducing organic molecules according to the invention may, for example, be a transfection process, where it is desired to transfer genetic information from a vector, such as a virus particle, into a target cell.

The target cells may be any procaryotic or eucaryotic cells and they may be of human or animal origin. Examples of target cells are any hematopoietic cells, stem cells, T-cells etc. Target cells may be used free or immobilized on suitable microcarriers, as mentioned above.

In one particular embodiment of the present invention, the method defined above also comprises a step for the production of particles containing organic molecules to be introduced in target cells. This may for example be especially advantageous in the case where the particles are some kind of rapidly degrading biomolecules, such as retroviruses. Accordingly, the method may also involve a step prior to the step of introducing organic molecules into target cells, comprising the culture of virus producing cells. Such a culture is performed according to any suitable process. Preferably, those cells will secrete the virus into the culture medium, which will constitute the liquid flow. If requisite, the virus producing cells may be separated from the virus containing liquid flow, or supernatant, in a separate step, e.g. by the use of a filter, before the liquid flow contacts the target cells. In a preferred embodiment of the method of the invention, the liquid flow is brought to pass through a layer or bed of target cells by a pumping means, such as a peristaltic pump, but, naturally, any kind of driving device which is capable of transporting the liquid flow in the desired direction is of use to this end. In addition or alternatively, a filter may also be used to remove other undesired material from the liquid flow, such as toxic or in other ways harmful material. Such material may be the cells producing the biomolecules themselves or other substances resulting from the production step.

In a third aspect the present invention relates to a system suitable for the performance of a method of introducing organic molecules into target cells, which comprises
- a reaction vessel adapted to contain said target cells and
- means for passing a liquid flow, which comprises particles containing said organic molecules, by said target cells, which preferably are present in a suspension or solution. Most preferably, said means for passage of a liquid flow through target cells comprises appropriate tubing as well as a pumping device and provides essentially constant flow conditions in the reaction vessel. In addition, a contact between said particles and said target cells is provided, which is sufficient to enable exit of the organic molecules from the particles and entrance thereof into the target cells. In this context, the term "essentially constant flow" should be interpreted as a flow, which is constant over a period of time, which, however, may contain shorter periods of intermittent flow or slightly irregular flow.

In this context, the used target cells, the particles comprising the organic molecules as well as the organic molecules themselves are as described in detail above in connection with the method according to the invention.

In one embodiment of the system according to the invention, the reaction vessel is a column. The choice of appropriate size and material thereof is easily made by someone skilled in this field considering the kind of process which is to be performed in the system.

In the most preferred embodiment of the system according to the invention, said means for passage of the liquid flow through the target cells provides a direction of the flow, which essentially counteracts the gravitational force, or any suitable multiplicity thereof, of the target cells. Alternatively, the liquid flow may counteract a centrifugal force applied on the target cells. In any case, the target cells are preferably free floating in the liquid as in a fluidized bed. In an alternative embodiment, the target cells are immobilized on one or more carriers, such as microcarriers, in which case the system provides a fluidized bed comprised of free floating microcarriers instead of free floating target cells.

In a particular embodiment of the system according to the invention, there are also provided means for recirculation of the liquid flow through the target cells to enhance the efficacy of the process performed therein.

Optionally, the system according to the invention may further comprise means for the production of said particles containing the organic molecules, such as a container, vessel or fermenter suitable for culture of the cells producing organic molecules as defined above, e.g. cells producing retroviruses. Conditions for such a culture are commonly known and may easily be adapted for each case by someone skilled in the art. In some cases, it may be advantageous to remove undesired material resulting from such a production step, in which case the system further comprises means for the separation thereof, such as a filter. Undesired material may e.g. be material which is toxic for the target cells or harmful in later application of target cells harbouring organic molecules.

### Detailed description of the drawings

Figure 1 discloses an advantageous apparatus for performing the method according to the invention. The retrovirus producing cells are cultivated in the medium/supernatant and or producer cell vessel (4) and the supernatant is pumped (3) through the cell separation filter (2), which prevents virus producing cells from contaminating the target cells, into the target cell chamber (1). The flow of the supernatant is low enough to prevent the target cells to be swept away but high enough to permit circulation in the target cell chamber to enable maximum exposure of virus particles to target cells. The producer cells in the reaction chamber can preferably be cultivated on microcarriers to allow for maximum retroviral titer in the supernatant. Target cells in the reaction chamber may grow in suspension or on microcarriers. The system is closed therby diminishing the risk of contamination by exogenous agents.The system can also be used for cell-free virussupernatants obviating the need for a cell separation filter. This system also allows for non-viral applications, such as transfections and biomolecule interactions with target cells.

In one preferred embodiment of the invention, a widening on the top of the reactor is provided to slow down the flow of supernatant enough so that no cells can escape.

When performing a method according to the invention with the disclosed equipment, the flow of supernatant should be low enough to prevent the target cells from being swept away from the reaction chamber 1, but high enough to permit circulation in the target cell chamber 1 to enable a maximal exposure of virus particles to target cells. The producer cell line in the producer cell vessel 4 is preferably cultivated on microcarriers to allow for a maximal retroviral titer in the supernatant. The disclosed system may alternatively be used for cell free virus supernatants, i.e. particle containing liquid flows, in which case the need of the cell separation filter (2) is obviated.

The present invention is more fully described below by an example, which is merely illustrating the invention and which is not to be interpreted as limiting the invention in any way.

### EXPERIMENTAL

The vessel that contains the supernatant or virus producer cell line is a flat bottomed 25 cm in diameter glass container. The surface does not need to be coated to allow for cell attachment. The opening is blocked by a 4 cm rubber cork with three openings:
1.) Outlet for the supernatant, 2.) Inlet for recirculating supernatant and 3.) A gas exchange filter.
   The minimum volume of medium that can be used in this vessel is approximately 150 ml. The outlet is mediated by a glass tube with an internal diameter of 3 mm and a height of 25 cm. This assures that any stray cells detaching from the bottom surface are incapable of entering the circulation, as the gravitational pull is stronger than the flow rate.
   The supernatant passes through a filter on its way to the reaction chamber. The filter consists of a filter holder with support screens and luer lock fittings. The membrane is a filter with diameter of 5 cm and a pore size of 45 µm. The membrane is a non-protein binding membrane and it is autoclavable. It is very important that the total assembly is autoclavable to ensure a sterile interior for the transduction. From the filter, the supernatant enters the reaction chamber, where the target cells are. The reaction chamber consists of a cylinder with an internal diameter of 1.5 cm and a height of 12 cm. This gives a volume of 22 cm³. There are two openings in the bottom of the reaction chamber. One is directly under the centre of the cylinder. This is where the supernatant enters. The other opening is at 90° angle to the reaction chamber. This is the opening where the target cells are injected into the reaction chamber and also extracted. The opening is fitted with a autoclavable rubber septum to simplify injection of the target cells. The septum is replaceable, since it is fitted with a screw adapter.
   The top of the reaction chamber has three openings:
   1. For injecting media to fill the reaction chamber before starting the circulation. This opening is fitted with a rubber septum. 2. To be used to level out internal air pressure during the filling of the reaction chamber. 3. The outlet where the supernatant exits after passing the reaction chamber.

All in/outlets for injecting medium or cells are fitted with Teflon faucets.
The outlet tube is connected via a outlet with a vault on it. It leeds straight to the medium container and enables recirculation of the medium/supernatant. The medium/supernatant vessel is placed on a stirrer table to allow for an equal distribution and also a homogenous concentration in the medium/supernatant circulating through the system. The peristaltic pump has a gear that allows for as low a pump rate as 10 ml/h. At this rate the cells within the reaction chamber will not escape it.

### Example 1: Retrovirus mediated gene transfer with retroviral supernatant

The system is put together and autoclaved. The supernatant is aliquoted so that the final concentration is allowing a moi that you choose. In this example, a moi of 5 is used. For a normal human bone marrow transplantation it is usual to use about 100 million, or more, nucleated cells or at least 10 million CD34⁺ cells. In this case, 10 million CD34⁺ cells are used. To achieve a moi of 5 50 million cfu are needed. If the supernatant has a concentration of 700 000, 71 ml of the original supernatant will be needed. This is supplemented with appropriate media and cytokines. The media for murine BMC's is Dulbecco's Modified Eagle Medium (DMEM). The cytokines used are IL-3, IL-6 and mSCF. For human cells the same medium and cytokines are used, however, with the difference that the murine cytokines are replaced with the human counterparts.

To fill the system approximately 15 ml of media will be needed, so when making the media, this amount is put a side. First the supernatant to be used is diluted with the freshly made DMEM and supplemented with the cytokines. Then polybrene or another cation of choice is added. In this system, a polybrene concentration of 4 µg/ml is used. The medium/supematant container is filled up with 200 ml of supernatant/media mix and put into the incubator together with the rest of the apparatus. To fill the system, the peristaltic pump is turned on and medium/supernatant is pumped until it enters the reaction chamber. After this is done, the reaction chamber is inoculated with the target cells, in this case 10 million CD34⁺ cells. The cells are injected through the lower opening with the rubber septum. The cells are injected in a volume of 2 ml. After injection of the cell suspension another 2 ml is injected to rinse away any stray cells from the vault and flush them into the reaction chamber. To fill the reaction chamber, 10-12 ml of supernatant/medium mix is slowly injected through the upper septum opening. The 10-12 ml are injected during about 2 minutes or more. Let the cells sediment for 30 minutes before starting the circulation. The circulation should be allowed to continue for as long as the cells are intended to be in the apparatus. For CD34⁺ cells, the cells are transduced for 48 hours. After the completion of the transduction period, the peristaltic pump isturned off. To extract the cells it is convenient to use a syringe and extract the contents of the reaction chamber. After this is done the peristaltic pump is started again and the flow is turned up to maximum, which in the present case is 35 ml/hour. This will cause all cells still remaining in the region just before the main reaction chamber to be flushed out into the reaction chamber. The reaction chamber is emptied again and the flushing procedure is repeated once more. The resulting 36 ml is centrifuged at 1500 rpm in a standard Sorvall centrifuge for 5 minutes and resuspended in PBS and spun again. After the last wash the cells are resuspended in 1 ml DMEM supplemented with cytokines and 20 µl is taken out for counting the number of surviving cells. For ordinary methylcellolose cultivation 1000 cells is needed for each 3 cm plate. The methylcellulose is supplemented with cytokines to optimise cell growth. The cells are also supplemented with 0.8 mg G-418/ml to select for cells positive for gene transfer. The assay in done in 5 copies and allowed to grow during 12-14 days and then evaluated for number of G-418 resistant colonies. There are also 3 copies of methylcellulose plates without G-418 to establish the total number of cells capable of forming colonies. The transduction efficacy is given in % resistant colonies v/s the number of colonies growing without G-418.

### Example 2: Retrovirus mediated gene transfer with retrovirus producer cell line

The system is put together and autoclaved. The medium for murine BMC's is Dulbecco's Modified Eagle Medium, DMEM. The cytokines used are IL-3, IL-6 and mSCF. The same medium is used for human CD34⁺ cells with the murine cytokines replaced with human counterparts. To fill the system, approximately 15 ml of medium is needed, so when making the media, this amount is put aside. First the media is supplemented with the cytokines. Then polybrene or another cation of choice is added. In this system a polybrene concentration of 4 µg/ml is used. The retrovirus producer cells is irradiated with 400 R and 500 000 cells is resuspended in the medium. The medium/supernatant container is filled up with 200 ml of cell suspension and put in to the incubator together with the rest of the apparatus. The cells are allowed to attach for 5 hours before preceding to fill the system. To fill the system the peristaltic pump is turned on and medium is pumped until it enters the reaction chamber. After this is done the reaction chamber is inoculated with the target cells, in this case 10 million CD34⁺ cells. The cells are injected through the lower opening covered by the rubber septum. The cells are injected in a volume of 2 ml. After injection of the cell suspension another 2 ml is injected to rinse away any stray cells from the vault and flush them into the reaction chamber. To fill the reaction chamber 10-12 ml of supernatant/medium mix is slowly injected through the upper opening. The 10-12 ml are injected slowly, for about 2 minutes or more. The cells are left to sediment for 30 minutes before starting the circulation. The circulation is allowed to continue for as long as the cells are intended to be in the apparatus. For CD34⁺ cells, the cells are transduced for +48 hours. After the transduction period is over the peristaltic pump isturned off. To extract the cells it is convenient to use a syringe and extract the contents of the reaction chamber. After this is done the peristaltic pump is started again and the flow is turned up to maximum, which in this case is 35 ml/hour. This will cause all cells still remaining in the region just before the main reaction chamber to be flushed out into the reaction chamber. The reaction chamber is emptied again and the flushing procedure is repeated once more. The resulting 36 ml is centrifuged at 1500 rpm in a standard Sorvall centrifuge for 5 minutes and resuspended in PBS and spun again. After the last wash the cells are resuspended in 1 ml DMEM supplemented with cytokines and 20 µl is taken out of for counting the number of surviving cells. For ordinary methylcellolose cultivation 1000 cells is needed for each 3 cm plate. The methylcellulose is supplemented with cytokines to optimise cell growth. The cells are also supplemented with 0.8 mg G-418/ml to select for cells positive for gene transfer. The assay in done in 5 copies and allowed to grow for 12-14 days and then evaluated for number of G-418 resistant colonies. There are also 3 copies of methylcellulose plates without G-418 to establish the total number of cells capable of forming colonies. The transduction efficacy is given in % resistant colonies v/s the number of colonies growing without G-418.

### Example 3: Reverse flow-through transduction (RFTT) apparatus used for non-viral cell targeting

In this example, CD34⁺ cells are transfected with a pEGFP™ vector using Superfect™

The system is put together and autoclaved. For the purpose of transfecting cells no filter should be used, since that would disrupt the DNA/superfect aggregates. The media for murine BMC's is Dulbecco's Modified Eagle Medium, DMEM. The cytokines used are IL-3, IL-6 and mSCF. The same media is used for human CD34⁺ cells with the murine cytokines replaced with human counterparts. If cells are used that demands a different medium this has to be used instead of DMEM. To fill the system approximately 15 ml of media is needed, so when making the media, this amount is put a side. The medium/supernatant container is filled up with 200 ml of medium and put in to the incubator together with the rest of the apparatus. To fill the system the peristaltic pump is turned on and medium is pumped until it starts to enter the reaction chamber. After this is done the reaction chamber is inoculated with the target cells, in this case 10 million CD34⁺ cells. The cells are injected through the lower opening coverd by the rubber septum. The cells are injected in a volume of _2 ml. After injection of the cell suspension another 2 ml is injected to rinse away any stray cells from the vault and flush them into the reaction chamber. To fill the reaction chamber 10-12 ml of supernatant/medium mix is slowly injected through the upper opening. The 10-12 ml are injected slowly, for about 2 minutes or more.Tthe cells are left to sediment for 30 minutes before starting the circulation. Before the circulation is started, the media is first supplemented with the cytokines, the plasmid is mixed with serum free DMEM, 200 µg with 6 ml serum free media. The mixture is added to the media/supernatant container and slowly swirled to allow mixing. After this the circulation is started. The circulation is allowed to continue for as long as the cells are intended to remain in the apparatus. After the transfection period is over the peristaltic pump is stopped. To extract the cells it is convenient to use a syringe and extract the contents of the reaction chamber. After this is done the peristaltic pump is started again and the flow is turned up to maximum in this case _35 ml/hour. This will cause all cells still remaining in the region just before the main reaction chamber to be flushed out into the reaction chamber. The reaction chamber is emptied again and the flushing procedure is repeated once more. The resulting 36 ml is centrifuged at 1500 rpm in a standard Sorvall centrifuge for 5 minutes and resuspended in PBS and spun again. After the last wash, the cells are resuspended in 1 ml DMEM supplemented with cytokines and 20 µl is taken out for counting the number of surviving cells. For ordinary methylcellolose cultivation 1000 cells is needed for each 3 cm plate. The methylcellulose is supplemented with cytokines to optimize cell growth. The cells are also supplemented with 0.8 mg G-418/ml to select for cells positive for gene transfer. The assay is done in 5 copies and allowed to grow for 72 hours to allow for maximum EGFP expression. There are also 3 copies of methylcellulose plates without G-418 to establish the total number of cells and assessing autofluorecence. The transfection efficacy is given in % green cells v/s the number of negative cells.

## Claims

1. A method of introducing organic molecules into target cells by means of a system according to claim 10, which comprises the step of passing a liquid flow, which comprises particles containing said organic molecules, through a target cell suspension or solution, **characterized in that** said liquid flow is controlled to provide essentially constant flow conditions between free floating particles and target cells and a direction of said liquid flow is provided which essentially counteracts the gravitational force of the target cells, and a centrifugal force applied on the target cells, establishing a sufficient contact area and time therebetween to enable the organic molecules to exit particles and enter target cells.

2. A method according to claim 1, wherein the same liquid flow is passed several times through the target cell suspension or solution.

3. A method according to any one of claims 1-2, which further comprises a step for the production of the particles, which contain organic molecules to be introduced into target cells.

4. A method according to any one of claims 1-3, which further comprises a step for separation of any undesired material from the liquid flow.

5. A method according to any one of claims 1-4, wherein the organic molecules are capable of interaction with cells.

6. A method according to claim 5, wherein said organic molecules are nucleic acids.

7. A method according to claim 5, wherein said organic molecules are virus.

8. A method according to claim 7, wherein said virus are retrovirus.

9. A method according to any one of claims 1-8, wherein the target cells are present in a suspension during the step of introduction of the organic molecules therein.

10. A system for the performance of a method of introducing organic molecules into target cells, which system comprises
- a reaction vessel (1) adapted to contain said target cells and
- means for passing a liquid flow comprising particles containing said organic molecules through a suspension or solution of said target cells,
**characterized in that** said means for passage of a liquid flow through the target cell suspension or solution comprises tubing and a pumping device (3) and provides essentially constant flow conditions in the reaction vessel (1) as well as a sufficiently close contact between said particles and said target cells to enable exit of the organic molecules from the particles and entrance thereof into the target cells, wherein said means for passage of a liquid flow provides a direction of the flow, which essentially counteracts the gravitational force of the target cells and a centrifugal force applied on the target cells.

11. A system according to claim 10, wherein the reaction vessel is a column.

12. A system according to any to claims 10 or 11, which further comprises means for recirculation of the liquid flow through the target cell suspension or solution.

13. A system according to any one of claims 10-12, which further comprises means for the production of said particles containing the organic molecules.

14. A system according to any one of claims 10-13, which further comprises means for the separation of any undesired material.

## Patentansprüche

1. Verfahren zur Einbringung organischer Moleküle in Zielzellen unter Verwendung eines Systems gemäß Anspruch 10, Schritte umfássend, bei denen man einen Flüssigkeitsstrom, der Partikel umfasst, welche die organischen Moleküle enthalten, durch eine Zielzellsuspension oder -lösung passieren lässt, wobei das Verfahren **dadurch gekennzeichnet ist, dass** der Flüssigkeitsstrom kontrolliert wird um im wesentlichen konstante Fliessbedingungen zwischen den frei fließenden Partikeln und den Zielzellen zu erreichen und eine Richtung des Flüssigkeitsstromes bereitgestellt wird, die im Wesentliche der Schwerkraft der Zielzellen entgegenwirkt, und eine zentrifugale Kraft auf die Zielzellen ausgeübt wird, wodurch eine ausreichende Kontaktfläche und Zeit zwischen diesen bereitgestellt wird, die es den organischen Molekülen ermöglicht, die Partikel zu verlassen und in die Zielzellen aufgenommen zu werden.

2. Verfahren gemäß Anspruch 1, bei dem derselbe Flüssigkeitsstrom mehrfach durch die Zielzellensuspension oder -lösung geführt wird.

3. Verfahren gemäß irgendeinem der Ansprüche 1 oder 2, ferner Schritte umfassend, bei denen man die Partikel, welche organische Moleküle enthalten, die in Zielzellen eingebracht werden sollen, herstellt.

4. Verfahren gemäß irgendeinem der Ansprüche 1 bis 3, das ferner einen Schritt zur Auftrennung von ungewünschtem Material aus dem Flüssigkeitsstrom umfasst.

5. Verfahren gemäß irgendeinem der Ansprüche 1 bis 4, bei dem die organischen Moleküle in der Lage sind, mit den Zellen zu interagieren.

6. Verfahren gemäß Anspruch 5, bei dem die organischen Moleküle Nukleinsäuren sind.

7. Verfahren gemäß Anspruch 5, bei dem die organischen Moleküle Viren sind.

8. Verfahren gemäß Anspruch 7, bei dem die Viren Retroviren sind.

9. Verfahren gemäß irgendeinem der Ansprüche 1 bis 8, bei dem die Zielzellen während der Aufnahme der organischen Moleküle in einer Suspension vorliegen.

10. Vorrichtung zur Durchführung eines Verfahrens zur Einbringung organischer Moleküle in Zielzellen, wobei die Vorrichtung umfasst:
- ein Reaktionsgefäß (1), das zur Aufnahme von Zielzellen ausgestaltet ist, und
- Mittel, mit denen der Flüssigkeitsstrom, welcher Partikel umfaßt, die organische Moleküle enthalten, durch eine Suspension oder Lösung der Zielzellen geführt werden kann,
**dadurch gekennzeichnet, dass** das Mittel, mit dem der Flüssigkeitsstrom durch die Zielzellsuspension oder -lösung passiert wird, eine Rohr- und eine Pumpvorrichtung (3) umfasst, und im Wesentlichen konstante Fließbedingungen in dem Reaktionsgefäß (1) erzeugt sowie einen ausreichend nahen Kontakt zwischen den Partikeln und den Zielzellen, um es den organischen Molekülen zu ermöglichen, die Partikel zu verlassen und in die Zielzellen aufgenommen zu werden, wobei das Mittel zur Führung des Flüssigkeitsstromes eine Flußrichtung vorgibt, die im Wesentlichen der Schwerkraft der Zielzellen entgegenwirkt, und eine Zentrifugalkraft auf die Zielzellen ausgeübt wird.

11. System gemäß Anspruch 10, bei dem das Reaktionsgefäß eine Säule ist.

12. System gemäß irgendeinem der Ansprüche 10 oder 11, das ferner Mittel umfasst, die eine Rezirkulation des Flüssigkeitsstromes durch die Zielzelllsuspension oder -lösung erlauben.

13. System gemäß irgendeinem der Ansprüche 10 bis 12, das ferner Mittel für die Herstellung der Partikel umfasst, welche die organischen Moleküle enthalten.

14. System gemäß irgendeinem der Ansprüche 10 bis 13, das ferner Mittel zur Auftrennung von irgendeinem ungewünschten Material umfasst.

## Revendications

1. Procédé d'introduction de molécules organiques dans des cellules cibles au moyen d'un système selon la revendication 10, qui comprend l'étape de faire passer un flux de liquide, qui comprend des particules contenant lesdites molécules organiques, à travers une suspension ou une solution de cellules cibles, **caractérisé en ce que** ledit flux de liquide est contrôlé de manière à fournir des conditions de flux essentiellement constantes entre les particules flottant librement et les cellules cibles et une direction dudit flux de liquide est fournie qui compense essentiellement la force de gravitation des cellules cibles, et une force centrifuge appliquée aux cellules cibles, créant une surface et une durée de contact suffisantes entre celles-ci pour permettre aux molécules organiques de quitter les particules et de pénétrer dans les cellules cibles.

2. Procédé selon la revendication 1, dans lequel le même flux de liquide passe plusieurs fois à travers la suspension ou la solution de cellules cibles.

3. Procédé selon l'une quelconque des revendications 1 à 2, qui comprend en outre une étape pour la production des particules qui contiennent les molécules organiques à introduire dans les cellules cibles.

4. Procédé selon l'une quelconque des revendications 1 à 3, qui comprend en outre une étape pour la séparation de toute matière indésirable du flux de liquide.

5. Procédé selon l'une quelconque des revendications 1 à 4, dans lequel les molécules organiques sont capables d'interaction avec les cellules.

6. Procédé selon la revendication 5, dans lequel lesdites molécules organiques sont des acides nucléiques.

7. Procédé selon la revendication 5, dans lequel lesdites molécules organiques sont des virus.

8. Procédé selon la revendication 7, dans lequel lesdits virus sont des rétrovirus.

9. Procédé selon l'une quelconque des revendications 1 à 8, dans lequel les cellules cibles sont présentes dans une suspension pendant l'étape d'introduction des molécules organiques dans celle-ci.

10. Système pour la mise en oeuvre d'un procédé d'introduction de molécules organiques dans des cellules cibles, lequel système comprend
- un réacteur (1) appropriée pour contenir lesdites cellules cibles et
- un moyen pour faire passer un flux de liquide comprenant les particules contenant lesdites molécules organiques à travers une suspension ou une solution desdites cellules cibles,
**caractérisé en ce que** ledit moyen de passage d'un flux de liquide à travers la suspension ou la solution des cellules cibles comprend des canalisations et un dispositif de pompage (3) et offre des conditions de flux essentiellement constantes dans la cuve de réaction (1) ainsi qu'un contact suffisamment étroit entre lesdites particules et lesdites cellules cibles pour permettre la sortie des molécules organiques des particules et la pénétration de celles-ci dans les cellules cibles, dans lequel ledit moyen de passage d'un flux de liquide fournit une direction du flux, qui compense essentiellement la force de gravitation des cellules cibles et une force centrifuge appliquée aux cellules cibles.

11. Système selon la revendication 10, dans lequel le réacteur est une colonne.

12. Système selon l'une quelconque des revendications 10 ou 11, qui comprend en outre un moyen pour la recirculation du flux de liquide à travers la suspension ou la solution de cellules cibles.

13. Système selon l'une quelconque des revendications 10 à 12, qui comprend en outre un moyen pour la production desdites particules contenant les molécules organiques.

14. Système selon l'une quelconque des revendications 10 à 13, qui comprend en outre un moyen pour la séparation de toute matière indésirable.
